# EUROPÄISCHE PATENTSCHRIFT

(11) **EP 0 967 200 B1**
(45) Veröffentlichungstag und Bekanntmachung des Hinweises auf die Patenterteilung: **24.04.2002**
(21) Anmeldenummer: 99111837.3
(22) Anmeldetag: 19.06.1999
(51) Int. Cl.: C07C 309/12, C07C 233/40, C07C 69/618, C07C 309/58, C07C 309/59, C07C 69/734, A61K 7/42

(54) **4,4-Diarylbutadiene als wasserlösliche photostabile UV-Filter für kosmetische und pharmazeutische Zubereitungen**
4,4-Diarylbutadienes as water soluble, photostable UV-filters for cosmetic and pharmaceutical preparations
4,4-Diarylbutadiènes comme filtres UV hydrosolubles et photostables pour préparations cosmétiques et pharmaceutiques

(30) Priorität: 26.06.1998 DE 19828463
(43) Veröffentlichungstag der Anmeldung: 29.12.1999
(73) Patentinhaber: BASF AKTIENGESELLSCHAFT, 67056 Ludwigshafen (DE)
(72) Erfinder: Habeck,Thorsten,Dr., 67149 Meckenheim (DE); Westenfelder, Horst, 67435 Neustadt (DE); Wünsch, Thomas, Dr., 67346 Speyer (DE)

(56) Entgegenhaltungen:
- WO-A-91/11989
- US-A- 4 788 206
- US-A- 4 950 467

## Beschreibung

Die Erfindung betrifft die Verwendung von 4,4-Diarylbutadienen als wasserlösliche photostabile UV-Filter in kosmetischen und pharmazeutischen Zubereitungen zum Schutz der menschlichen Epidermis oder menschlicher Haare gegen UV-Strahlung, speziell im Bereich von 320 bis 400 nm.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für bestimmte kosmetische Präparate noch eine Vielzahl weiterer Anforderungen erfüllen, beispielsweise gute Löslichkeit in Wasser oder in mit Wasser mischbaren Lösungsmittel, wie z.B. Alkoholen, hohe Stabilität der mit ihnen hergestellten Lösungen oder Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoyimethan als UV-A-Filter beschrieben. Es wird vorgeschlagen, diesen speziellen UV-A-Filter, der von der Firma GIVAUDAN unter der Bezeichnung "PAR-SOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

US 4,950,467 beschreibt die Verwendung von 2,4-Pentadiensäurederivaten als UV-Absorber in kosmetischen Präparaten. Die in dieser Patentschrift bevorzugt genannten Monoaryl-substituierten Verbindungen haben ebenfalls den Nachteil, daß sie nicht genügend photostabil sind.

Es bestand daher die Aufgabe, Lichtschutzmittel für kosmetische und pharmazeutische Zwecke vorzuschlagen, die im UV-A-Bereich mit hoher Extinktion absorbieren, die photostabil sind, eine geringe Eigenfarbe d.h. eine scharfe Bandenstrukur aufweisen und gut in Wasser oder in mit Wasser mischbaren Lösungsmitteln löslich sind.

Diese Aufgabe wurde erfindungsgemäß gelöst durch Verwendung von 4,4-Diarylbutadienen der Formel I 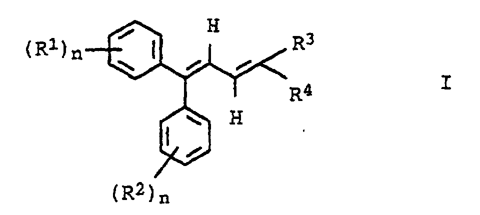 in der das Diensystem in der Z,Z; Z,E; E,Z oder E,E Konfiguration oder einer Mischung davon vorliegt und in der die Variablen unabhängig voneinander folgende Bedeutung haben:
- R¹ und R²: Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Heteroaryl, Aryl, das gegebenenfalls mit einem oder mehreren Resten wie Halogen, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein kann,
wasserlöslich machende Substituenten, ausgewählt aus der Gruppe bestehend aus Carboxylat-, Sulfonat- oder Ammoniumresten;
- R³: Wasserstoff, COOR⁵, COR⁵, CONR⁵R⁶, CN;
- R⁴: COOR⁶, COR⁶, CONR⁵R⁶;
- R⁵: Wasserstoff, [X]ₒ-R⁷, C₁-C₆-Alkylen-SO₃Y, C₁-C₆-Alkylen-PO₃Y, C₁-C₆-Alkylen-N(R⁸)₃⁺ A⁻;
- R⁶: [X]ₒ-R⁷, C₁-C₆-Alkylen-SO₃Y, C₁-C₆-Alkylen-PO₃Y, C₁-C₆-Alkylen-N(R⁸)₃⁺ A⁻;
- X: -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
- A: Cl, Br, J, SO₄R⁹;
- Y: Wasserstoff, Na⁺, K⁺, Mg²⁺, Ca²⁺, Li⁺, Al³⁺, N(R⁸)₄⁺;
- Z: O, NH;
- R⁷ und R⁸: Wasserstoff, C₁-C₆-Alkyl, C₂-C₆-Alkenyl, C₁-C₆-Acyl;
- R⁹: Wasserstoff, C₁-C₆-Alkyl, C₂-C₆-Alkenyl;
- n: 1 bis 3;
- o: 1 bis 150
als wasserlösliche photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

Als Alkylreste R¹ und R² seien verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, l-Ethyl-l-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl genannt.

Als Alkenylreste R¹ und R² seien verzweigte oder unverzweigte C₂-C₁₀-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl, 2-Hexenyl, 1-Heptenyl, 2-Heptenyl, 1-Octenyl oder 2-Octenyl genannt.

Als Cycloalkylreste seien für R¹ und R² bevorzugt verzweigte oder unverzweigte C₃-C₁₀-Cycloalkylreste wie Cyclopropyl, Cyclobutyl, Cyclopentyl, Cyclohexyl, Cycloheptyl, 1-Methylcyclopropyl, 1-Ethylcyclopropyl, 1-Propylcyclopropyl, 1-Butylcyclopropyl, 1-Pentylcyclopropyl, l-Methyl-l-Butylcyclopropyl, 1,2-Dimethylcyclopropyl, 1-Methyl-2-Ethylcyclopropyl, Cyclooctyl, Cyclononyl oder Cyclodecyl genannt.

Als Cycloalkenylreste seien für R¹ und R² bevorzugt verzweigte oder unverzweigte, C₃-C₁₀-Cycloalkenylreste mit einer oder mehreren Doppelbindungen wie Cyclopropenyl, Cyclobutenyl, Cyclopentenyl, Cyclopentadienyl, Cyclohexenyl, 1,3-Cyclohexadienyl, 1,4-Cyclohexadienyl, Cycloheptenyl, Cycloheptatrienyl, Cyclooctenyl, 1,5-Cyclooctadienyl, Cyclooctatetraenyl, Cyclononenyl oder Cyclodecenyl genannt.

Die Cycloalkenyl- und Cycloalkylreste können ggf. mit einem oder mehreren, z.B. 1 bis 3 Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein oder 1 bis 3 Heteroatome wie Schwefel, Stickstoff, dessen freie Valenzen durch Wasserstoff oder C₁-C₄-Alkyl abgesättigt sein können oder Sauerstoff im Ring enthalten.

Als Alkoxyreste für R¹ und R² kommen solche mit 1 bis 12 C-Atomen, vorzugsweise mit 1 bis 8 C-Atomen in Betracht.

Beispielsweise sind zu nennen:

| | |
|---|---|
| Methoxy- | Ethoxy- |
| Isopropoxy- | n-Propoxy- |
| 1-Methylpropoxy- | n-Butoxy- |
| n-Pentoxy- | 2-Methylpropoxy- |
| 3-Methylbutoxy- | 1,1-Dimethylpropoxy- |
| 2,2-Dimethylpropoxy- | Hexoxy- |
| l-Methyl-l-ethylpropoxy- | Heptoxy- |
| Octoxy- | 2-Ethylhexoxy- |

Alkoxycarbonylreste für R¹ und R² sind z.B Ester, die die oben genannten Alkoxyreste oder Reste von höheren Alkoholen z.B. mit bis zu 20 C-Atomen, wie iso-C₁₅-Alkohol, enthalten.

Als Mono- oder Dialkylaminoreste für R¹ und R² kommen solche in Betracht, die Alkylreste mit 1 bis 12 C-Atomen enthalten, wie z.B. Methyl-, n-Propyl-, n-Butyl-, 2-Methylpropyl-, 1,1-Dimethylpropyl-, Hexyl-, Heptyl-, 2-Ethylhexyl-, Isopropyl-, 1-Methylpropyl-, n-Pentyl-, 3-Methylbutyl-, 2,2-Dimethylpropyl-, 1-Methyl-1-ethylpropyl- und Octyl.

Unter Aryl sind aromatische Ringe oder Ringsysteme mit 6 bis 18 Kohlenstoffatomen im Ringsystem zu verstehen, beispielsweise Phenyl oder Naphthyl, die ggf. mit einem oder mehreren Resten wie Halogen z.B. Fluor, Chlor oder Brom, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein können. Bevorzugt sind ggf. substituiertes Phenyl, Methoxyphenyl und Naphthyl.

Heteroaryl-Reste sind vorteilhafterweise einfache oder kondensierte aromatische Ringsysteme mit einem oder mehreren heteroaromatischen 3- bis 7-gliedrigen Ringen. Als Heteroatome können ein oder mehrere Stickstoff-, Schwefel- und/oder Sauerstoffatome im Ring oder Ringsystem enthalten sein.

Hydrophile d.h. die Wasserlöslichkeit der Verbindungen der Formel I ermöglichende Reste für R¹ und R² sind z.B. Carboxy- und Sulfoxyreste und insbesondere deren Salze mit beliebigen physiologisch verträglichen Kationen, wie die Alkalisalze oder wie die Trialkylammoniumsalze, wie Tri-(hydroxyalkyl)-ammoniumsalze oder die 2-Methylpropan-1-ol-2-ammoniumsalze. Ferner kommen Ammonium-, insbesondere Alkylammoniumreste mit beliebigen physiologisch verträglichen Anionen in Betracht.

Als Alkylengruppen für R⁵ und R⁶, die hydrophile SO₃Y, PO₃Y oder N(R⁸)₃⁺A⁻ Reste tragen, seien verzweigte oder unverzweigte C₁-C₆-Alkylenreste, bevorzugt Methylen, Ethylen, n-Propylen, 1-Methylethylen, n-Butylen, 1-Methylpropylen, 2-Methylpropylen, n-Pentylen oder n-Hexylen genannt.

Der Rest [X]ₒ-R⁷ für R⁵ und R⁶ steht u.a. für Polyalkylenglykole, die aus 1 bis 150 Monomereinheiten, bevorzugt 1 bis 50, besonders bevorzugt aus 1 bis 30 Monomereinheiten zusammengesetzt sein können. X kann dabei eine Alkylenglykol-Monomereinheit sein, ausgewählt aus der Gruppe, bestehend aus Ethylenglykol, n-Propylenglykol, 1-Methylethylenglykol, n-Butylenglykol und 1-Ethylethylenglykol. Bevorzugte Monomereinheiten seien Ethylenglykol, n-Propylenglykol und 1-Methylethylenglykol.

Die Polyalkylenglykole können endständig alkyliert, alkenyliert oder acyliert sein.

Als Alkylreste für R⁷ bis R⁹ seien verzweigte oder unverzweigte C₁-C₆-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl oder 1-Ethyl-2-methylpropyl genannt.

Als Alkenylreste für R⁷ bis R⁹ seien verzweigte oder unverzweigte C₂-C₆-Alkenylketten, bevorzugt Vinyl, Propenyl, Isopropenyl, 1-Butenyl, 2-Butenyl, 1-Pentenyl, 2-Pentenyl, 2-Methyl-1-butenyl, 2-Methyl-2-butenyl, 3-Methyl-1-butenyl, 1-Hexenyl oder 2-Hexenyl genannt.

Als Acylreste für R⁷ und R⁸ seien C₁-C₆-Acylreste, bevorzugt Formyl, Acetyl, Propionyl oder n-Butyryl genannt.

Für [X]ₒ-R⁷ können auch Polyalkylenpolyamine, insbesondere Polyethylenimine in Betracht kommen, die ebenfalls aus 1 bis 150 Monomereinheiten, bevorzugt 1 bis 50, besonders bevorzugt aus 1 bis 30 Monomereinheiten zusammengesetzt sein können.

Bevorzugt sind solche Verbindungen der Formel I, in der
- R¹ und R²: unabhängig voneinander Wasserstoff, C₁-C₈-Alkyl, C₁-C₈-Alkoxy, C₁-C₈-Alkylamino, C₁-C₈-Dialkylamino,
wasserlöslich machende Substituenten, ausgewählt aus der Gruppe bestehend aus Carboxylat-, Sulfonat- oder Ammoniumresten;
- R³: COOR⁵, CONR⁵R⁶, CN;
- R⁴: COOR⁶, CONR⁵R⁶;
- R⁵: Wasserstoff, [X]ₒ-R⁷, C₁-C₆-Alkylen-SO₃Y, C₁-C₆-Alkylen-N(R⁸)₃⁺ A⁻;
- R⁶: [X]ₒ-R⁷, C₁-C₆-Alkylen-SO₃Y, C₁-C₆-Alkylen-N(R⁸)₃⁺ A⁻;
- X: -CH₂-CH₂-O-, -CH₂-CH₂-CH₂-O-, -CH(CH₃)-CH₂-O-;
- A: Cl, Br, J, SO₄R⁹;
- Y: Wasserstoff, Na⁺, K⁺, Mg²⁺, Ca²⁺, Li⁺, Al³⁺, N(R⁸)₄⁺;
- R⁷ und R⁹: Wasserstoff, C₁-C₃-Alkyl;
- n: 1 bis 3;
- o: 1 bis 50
bedeutet.

Als C₁-C₈-Alkylreste seien für R¹ und R² besonders bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 2-Ethylhexyl genannt.

Als Alkoxyreste für R¹ und R² kommen bevorzugt solche mit 1 bis 8 C-Atomen, besonders bevorzugt solche mit 1 bis 4 C-Atomen in Betracht.

Beispielsweise sind zu nennen:

| | |
|---|---|
| Methoxy- | Ethoxy- |
| Isopropoxy- | n-Propoxy- |
| n-Butoxy- | 1-Methylpropoxy- |
| 2-Methylpropoxy- | |

Als Mono- oder Dialkylaminoreste kommen für R¹ and R² besonders bevorzugt Methyl-, Ethyl-, n-Propyl-, n-Butyl-, 2-Methylpropyl-, 1,1-Dimethylpropyl-, 2-Ethylhexyl in Betracht.

Als besonders bevorzugte C₁-C₃-Alkylreste seien für R⁷ bis R⁹ Methyl, Ethyl, n-Propyl oder iso-Propyl genannt.

Als besonders bevorzugte Alkylengruppen für R⁵ und R⁶, die hydrophile SO₃Y, PO₃Y oder N(R⁸)₃⁺A⁻ Reste tragen, seien Methylen, Ethylen, n-Propylen, 1-Methylethylen oder n-Butylen genannt.

Die Substituenten R¹ und R² können jeweils in ortho, meta und/oder para Position am Aromaten gebunden sein. Im Falle von disubstituierten Aromaten (n = 2) können R¹ und R² in ortho/para oder meta/para Position vorliegen. Bevorzugt sind Verbindungen der Formel I mit n = 1, in denen R¹ gleich R² ist und beide Reste in der para-Position vorliegen.

Weiterhin weisen Verbindungen der Formel I (n = 1) besondere photostabile Eigenschaften aus, bei denen die Substituenten R¹ bis R⁴ in der in Tabelle 1 genannten Kombination vorliegen:

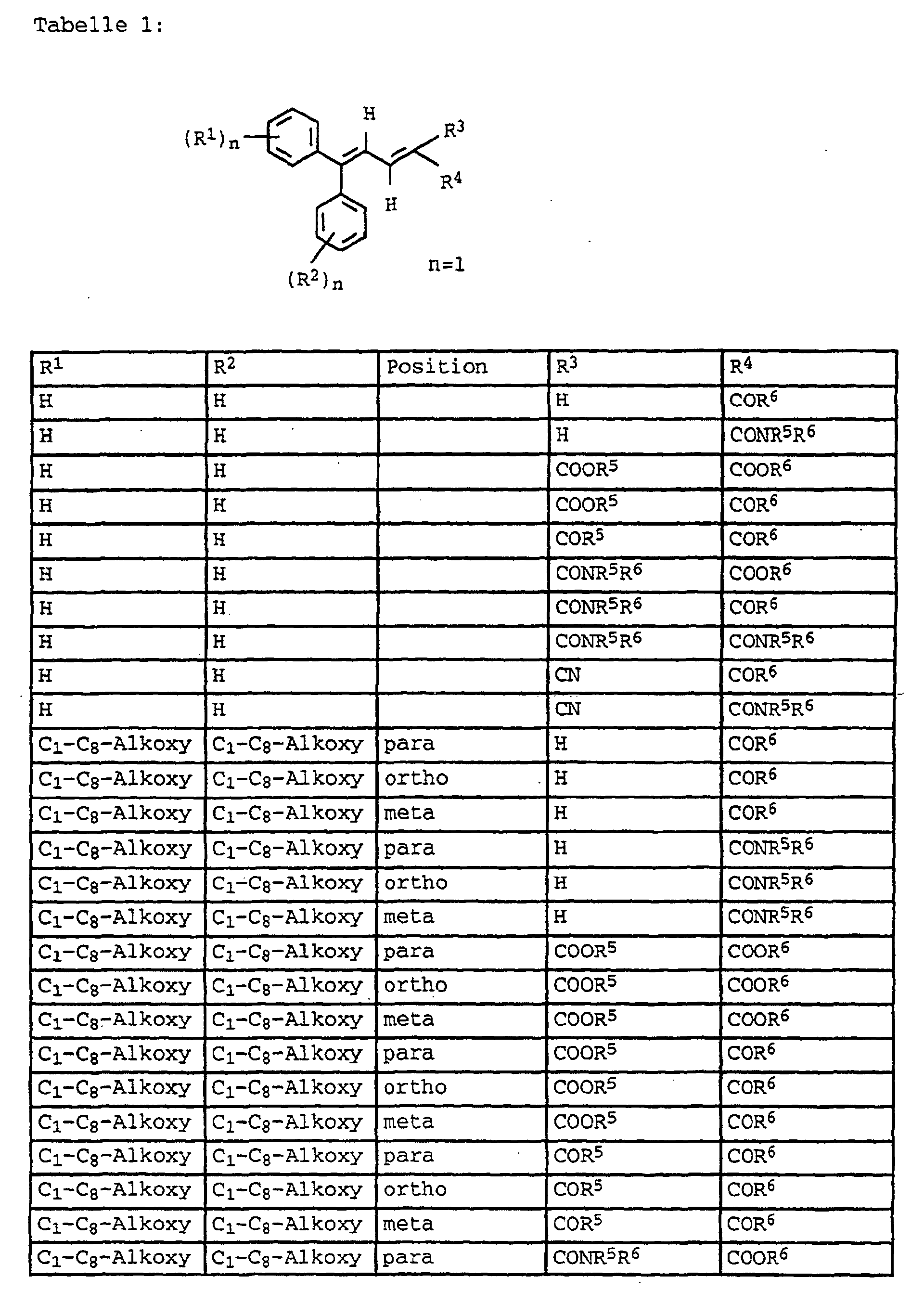

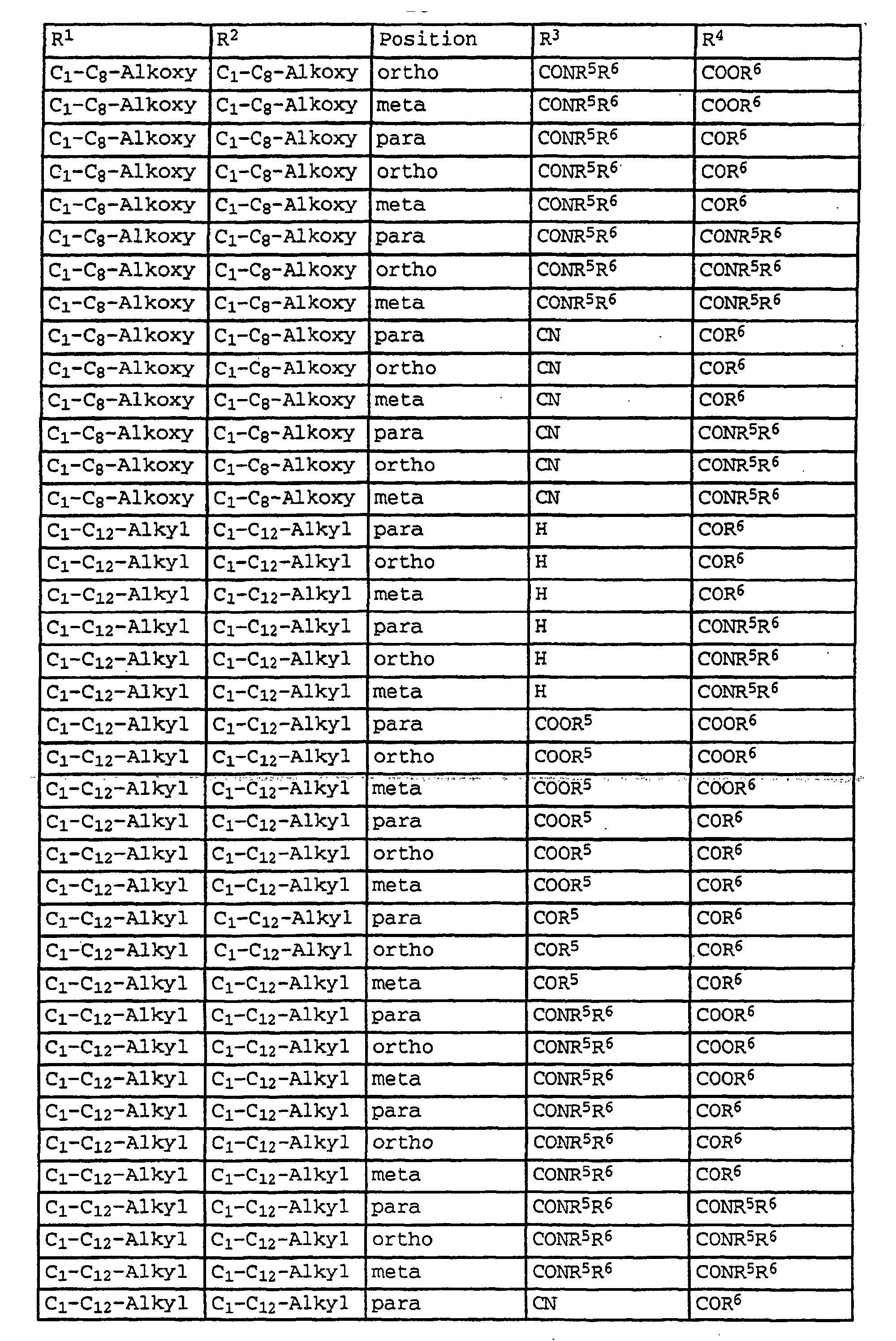

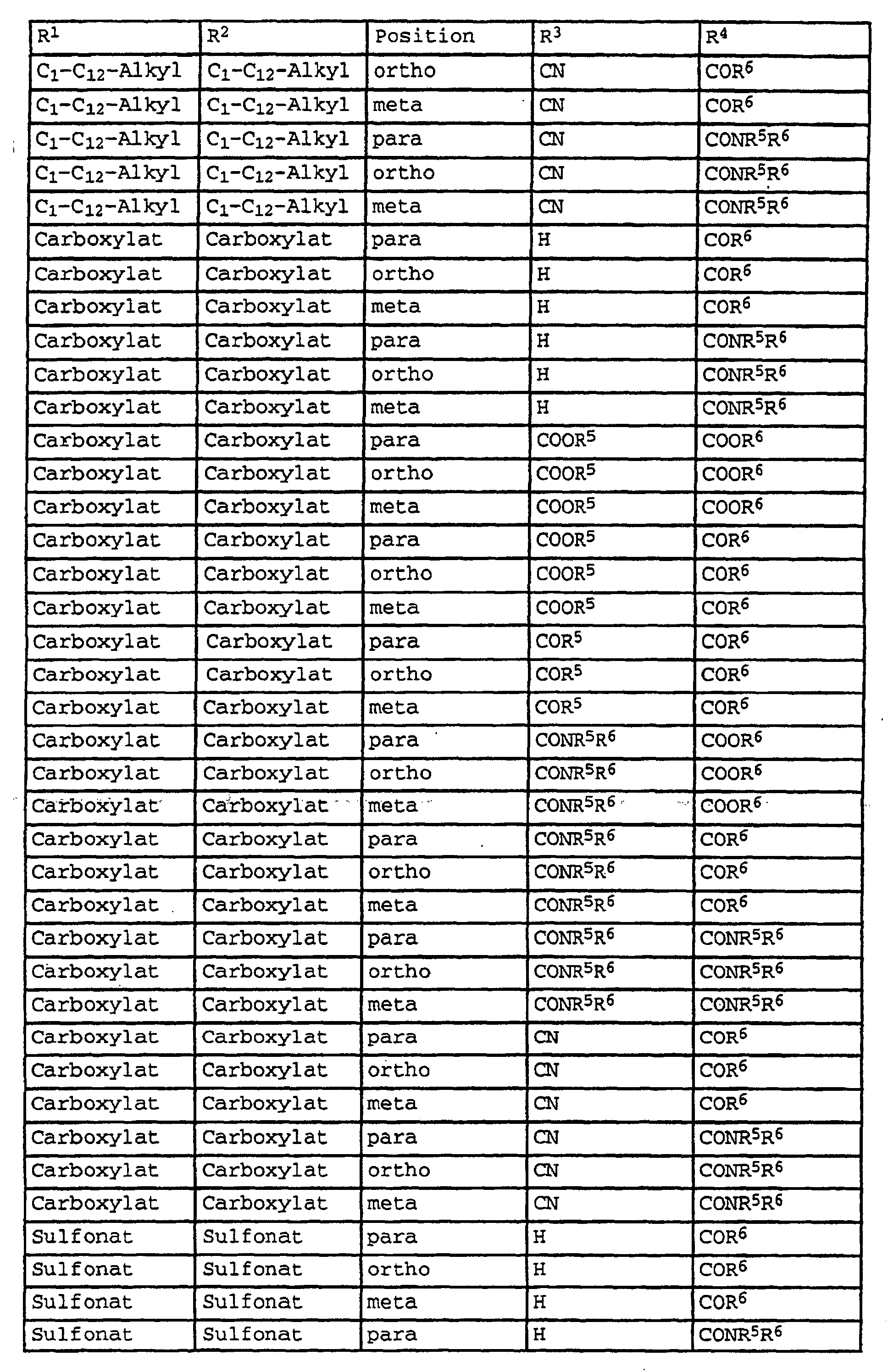

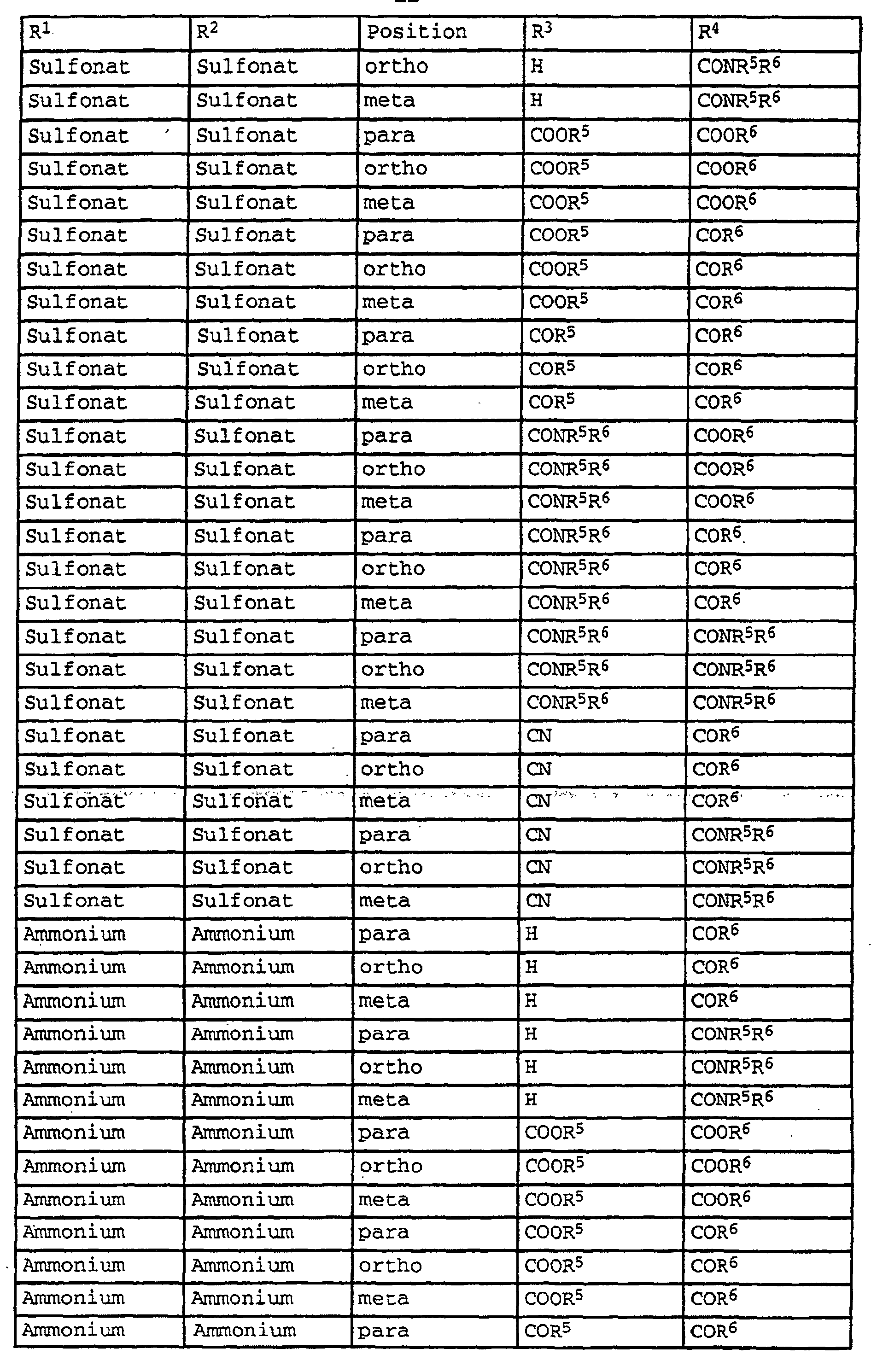

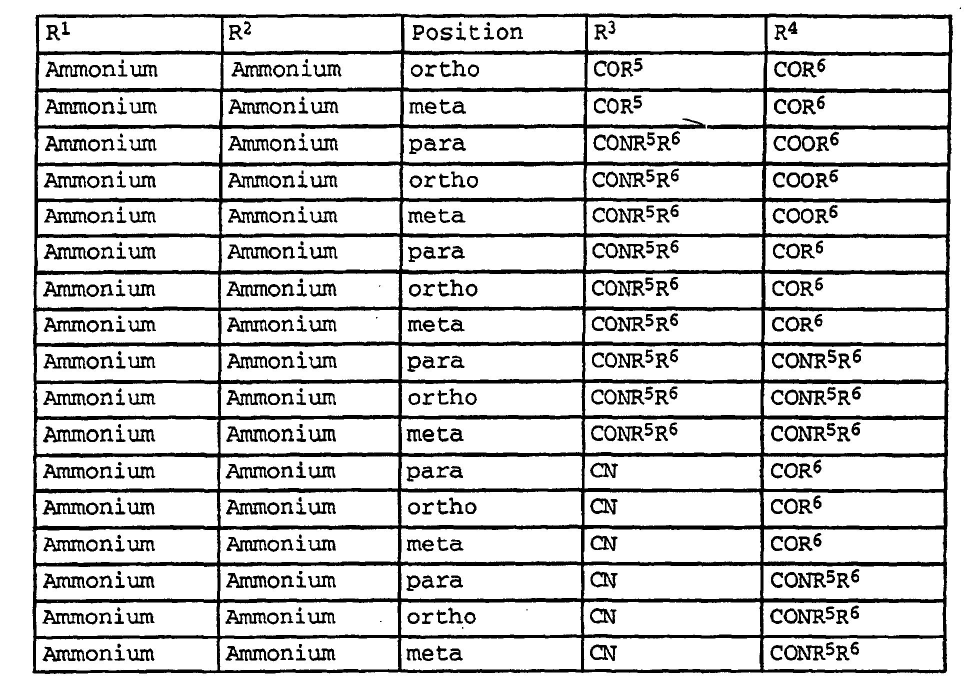

Die Erfindung betrifft auch 4,4-Diarylbutadiene der Formel I, 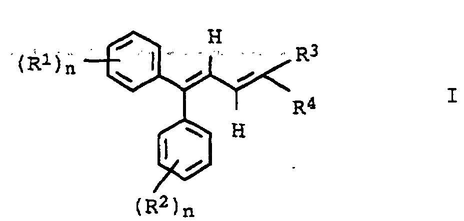 in der das Diensystem in der Z,Z; Z,E; E,Z oder E,E Konfiguration oder einer Mischung davon vorliegt und in der die Variablen unabhängig voneinander folgende Bedeutung haben:
- R¹ und R²: Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Heteroaryl, Aryl, das gegebenenfalls mit einem oder mehreren Resten wie Halogen, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein kann,
wasserlöslich machende Substituenten, ausgewählt aus der Gruppe bestehend aus Carboxylat-, Sulfonat- oder Ammoniumresten;
- R³: Wasserstoff, COOR⁵, COR⁵, CONR⁵R⁶, CN;
- R⁴: COOR⁶, COR⁶, CONR⁵R⁶ ;
- R⁵: Wasserstoff, [X]ₒ-R⁷, C₁-C₆-Alkylen-SO₃Y, C₁-C₆-Alkylen-PO₃Y, C₁-C₆-Alkylen-N(R⁸)₃⁺ A-;
- R⁶: [X]ₒ-R⁷, C₁-C₆-Alkylen-SO₃Y, C₁-C₆-Alkylen-PO₃Y, C₁-C₆-Alkylen-N(R⁸)₃⁺ A⁻;
- X: -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
- A: Cl, Br, J, SO₄R⁹;
- Y: Wasserstoff, Na⁺, K⁺, Mg²⁺, Ca²⁺, Li⁺, Al³⁺, N(R⁸)₄⁺;
- Z: O, NH;
- R⁷ und R⁸: Wasserstoff, C₁-C₆-Alkyl, C₂-C₆-Alkenyl, C₁-C₆-Acyl;
- R⁹: Wasserstoff, C₁-C₆-Alkyl, C₂-C₆-Alkenyl;
- n: 1 bis 3;
- o: 1 bis 150.

Bevorzugt sind 4,4-Diarylbutadiene der Formel Ia, 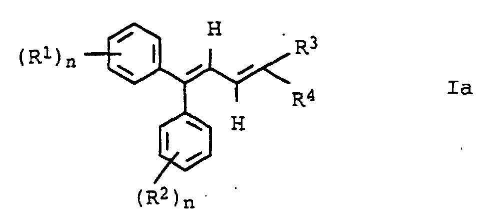 in der das Diensystem in der Z,Z; Z,E; E,Z oder E,E Konfiguration oder einer Mischung davon vorliegt und in der die Variablen unabhängig voneinander folgende Bedeutung haben:
- R¹ und R²: Wasserstoff, C₁-C₈-Alkyl, C₁-C₈-Alkoxy,
wasserlöslich machende Substituenten, ausgewählt aus der Gruppe bestehend aus Carboxylat-, Sulfonat- oder Ammoniumresten;
- R³: COOR⁵, CONR⁵R⁶, CN;
- R⁴: COOR⁶, CONR⁵R⁶;
- R⁵: Wasserstoff, [X]ₒ-R⁷, C₁-C₆-Alkylen-SO₃Y, C₁-C₆-Alkylen-N(R⁸)₃⁺ A⁻;
- R⁶: [X]ₒ-R⁷, C₁-C₆-Alkylen-SO₃Y, C₁-C₆-Alkylen-N(R⁸)₃⁺ A⁻;
- X: -CH₂-CH₂-O-, -CH₂-CH₂-CH₂-O-, -CH(CH₃)-CH₂-O-;
- A: Cl, Br, J, SO₄R⁹;
- Y: Wasserstoff, Na⁺, K⁺, Mg²⁺, Ca²⁺, Li⁺, Al³⁺, N(R⁸)₄⁺;
- R⁷ bis R⁹: Wasserstoff, C₁-C₃-Alkyl;
- n: 1 bis 3;
- o: 1 bis 50.

Besonders bevorzugt sind 4,4-Diarylbutadiene der Formel Ib, 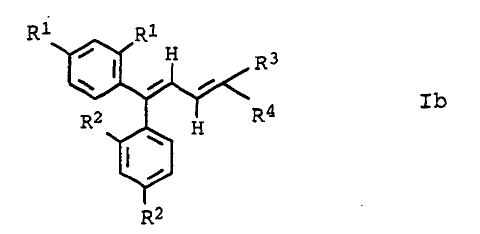 in der das Diensystem in der Z,Z; Z,E; E,Z oder E,E Konfiguration oder einer Mischung davon vorliegt und in der die Variablen unabhängig voneinander folgende Bedeutung haben:
- R¹ und R²: Wasserstoff, C₁-C₈-Alkyl, C₁-C₈-Alkoxy;
- R³: COOR⁵, CONR⁵R⁶, CN;
- R⁴: COOR⁶, CONR⁵R⁶;
- R⁵: Wasserstoff, [X]ₒ-R⁷, C₁-C₆-Alkylen-SO₃Y, C₁-C₆-Alkylen-N(R⁸)₃⁺ A⁻ ;
- R⁶: [X]ₒ-R⁷, C₁-C₆-Alkylen-SO₃Y, C₁-C₆-Alkylen-N(R⁸)₃⁺ A⁻;
- X: -CH₂-CH₂-O-, -CH₂-CH₂-CH₂-O-, -CH(CH₃)-CH₂-O-;
- A: Cl, Br, J, SO₄R⁹;
- Y: Wasserstoff, Na⁺, K⁺, Mg²⁺, Ca²⁺, Li⁺, Al³⁺, N(R⁸)₄⁺;
- R⁷ bis R⁹: Wasserstoff, C₁-C₃-Alkyl;
- o: 1 bis 50.

Ganz besonders bevorzugt sind 4,4-Diarylbutadiene der Formel Ic, 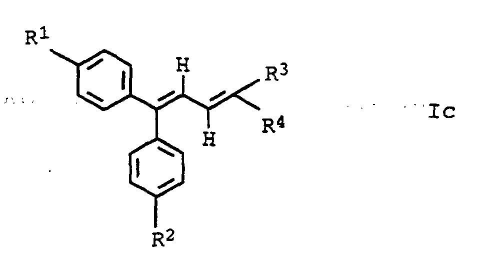 in der das Diensystem in der Z,Z; Z,E; E,Z oder E,E Konfiguration oder einer Mischung davon vorliegt und in der die Variablen unabhängig voneinander folgende Bedeutung haben:
- R¹ und R²: Wasserstoff, C₁-C₈-Alkyl, C₁-C₈-Alkoxy;
- R³: COOR⁵, CONR⁵R⁶, CN;
- R⁴: COOR⁶, CONR⁵R⁶;
- R⁵: Wasserstoff, [X]ₒ-R⁷, C₁-C₆-Alkylen-SO₃Y, C₁-C₆-Alkylen-N(R⁸)₃⁺ A⁻;
- R⁶: [X]ₒ-R⁷, C₁-C₆-Alkylen-SO₃Y, C₁-C₆-Alkylen-N(R⁸)₃⁺ A⁻;
- X: -CH₂-CH₂-O-, -CH₂-CH₂-CH₂-O-, -CH(CH₃)-CH₂-O-;
- A: Cl, Br, J, SO₄R⁹;
- Y: Wasserstoff, Na⁺, K⁺, Mg²⁺, Ca²⁺, Li⁺, Al³⁺, N(R⁸)₄⁺;
- R⁷ bis R⁹: Wasserstoff, C₁-C₃-Alkyl;
- o: 1 bis 50.

Die genauere Definition der Substituenten R¹ bis R⁹ der Verbindungen I sowie der bevorzugten Vertreter Ia bis Ic entspricht der bereits eingangs für die Verbindung I erfolgten Beschreibung.

Die erfindungsgemäß zu verwendenden Verbindungen der Formel I können nach der Gleichung 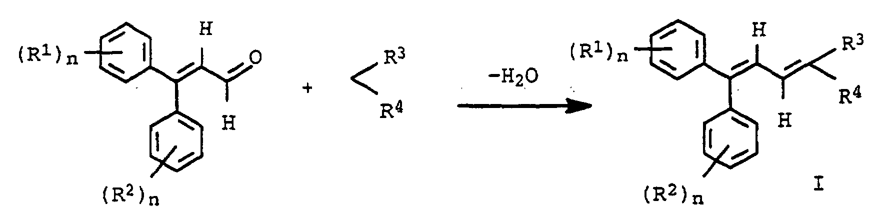 durch Kondensation hergestellt werden, wobei R¹ bis R⁴ die im Anspruch 1 genannte Bedeutung haben.

Die oben genannte Kondensation kann sowohl basen- als auch säurekatalysiert erfolgen. Geeignete Katalysatoren sind:
tertiäre Amine, wie z.B. Pyridin, Morpholin, Triethylamin, Triethanolamin;
sekundäre Amine, wie z.B. Piperidin, Dimethylamin, Diethylamin;
NH₃, NaNH₂, KNH₂, NH₄OAc;
basisches Aluminiumoxid, basischer Ionenaustauscher;
Na₂CO₃, K₂CO₃; saure Katalysatoren, wie z.B. Eisessig, Ameisensäure, Propionsäure;
HCl, H₂SO₄, HNO₃;
saurer Ionenaustauscher.

Die Menge der Katalysatoren beträgt im allgemeinen 0.1 bis 50 mol-%, bevorzugt 0.5 bis 20 mol-%, der Menge des eingesetzten Aldehyds.

Vorzugsweise arbeitet man bei Temperaturen von 20 bis 150°C, besonders 30 bis 100°C, besonders bevorzugt 40 bis 80°C. Besondere Bedingungen bezüglich des Druckes sind nicht erforderlich; im allgemeinen nimmt man die Umsetzung bei Atmosphärendruck vor.

Als Lösungsmittel können Alkohole, wie z.B. Methanol, Ethanol oder Isopropanol; Aromaten, wie z.B. Toluol oder Xylol; Kohlenwasserstoffe, beispielsweise Heptan oder Hexan; chlorierte Kohlenwasserstoffe, wie z.B. Chloroform oder Dichlormethan; Miglyol, Tetrahydrofuran eingesetzt werden. Die Reaktion kann aber auch ohne Lösungsmittel durchgeführt werden.

Es ist auch möglich, ausgehend von Methyl- oder Ethylestern, wie z.B. Verbindung 1 in Tabelle 2, längerkettige Ester durch Umesterungsreaktionen in Gegenwart eines basischen Katalysators herzustellen.

Für die Umesterung geeignete Katalysatoren sind:
basische Alkali- und Erdalkalisalze, bevorzugt solche, die weder in den Edukten noch in den Produkten löslich sind und sich nach Reaktionsende leicht abtrennen lassen, besonders bevoruzgt: Natrium-, Kalium- oder Calciumcarbonat oder Natriuumhydrogencarbonat;
Erdalkalioxide, bevorzugt Calcium- oder Magnesiumoxid und
basische Zeolithe.

Die Menge der Katalysatoren beträgt im allgemeinen 1 bis 80 mol-%, bevorzugt 5 bis 50 mol-%, der Menge des eingesetzten Esters.

Die Menge an eingesetzten Alkohol muß mindestens äquimolar sein zur eingesetzten Menge an Ausgangsester, beispielsweise Verbindung 1 in Tabelle 2. Bevorzugt werden Mengen von 200 bis 500 mol-% des Alkohols verwendet.

Die Entfernung des gebildeten Methanols oder Ethanols erfolgt destillativ.

Vorzugsweise arbeitet man bei Temperaturen von 50 bis 250°C, besonders 60 biis 150°C. Besondere Bedingungen bezüglich des Druckes sind nicht erforderlich; im allgemeinen nimmt man die Umsetzung bei Atmosphärendruck vor.

Als Lösungsmittel können inerte, höher siedende Verbindungen wie Xylole, aber auch Toluol oder Gemische der eingesetzten Alkohole mit flüssigen, kurzkettigen Alkanen wie Hexan und Heptan, eingesetzt werden. Bevorzugt arbeitet man lösungsmittelfrei in dem eingesetzten Alkohol.

Die Umesterung kann sowohl diskontinuierlich als auch kontinuierlich durchgeführt werden. Bei der kontinuierlichen Fahrweise leitet man die Reaktionspartner vorzugsweise über ein Festbett aus einer unlöslichen Base.

Für den Fall, daß R³ ≠ R⁴, können die erfindungsgemäßen Verbindungen der Formel I prinzipiell in ihren verschiedenen geometrischen Isomeren, d. h. mit einem Z,Z; Z,E; E,Z und/oder E,E-konfigurierten Diensystem, vorliegen. Bevorzugt als kosmetische Lichtschutzmittel sind die all-E- und/oder all-Z-Isomeren, ganz besonders bevorzugt sind die all-E-Isomeren.

Ist R³ = R⁴, so kann die C-C Doppelbindung zwischen C-3 und C-4 (in Nachbarstellung zum Diarylsystem) in der E- und/oder Z-Konfiguration, bevorzugt in der Z-Konfiguration vorliegen.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-A- und UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Die erfindungsgemäßen Verbindungen der Formel I lassen sich für die Herstellung der o.g. Sonnenschutzmittel vorteilhafterweise in die wäßrige bzw. alkoholisch-wäßrige Phase einarbeiten.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß- Heiß/Kalt- bzw. PIT-Emulgierung erfolgen. Hierbei handelt es sich um ein rein mechanisches Verfahren, eine chemische Reaktion findet nicht statt.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen:

**Tabelle 2:**

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon(Sulisobenzonum) und das Natriumsalz | 4065-45-6 |
| 13 | 3-(4'-Methyl)benzyliden-bornan-2-on | 36861-47-9 |
| 14 | 3-Benzylidenbornan-2-on | 15087-24-8 |
| 15 | 1-(4'-Isopropylphenyl)-3-phenylpropan-1,3-dion | 63250-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2', 4,4'-Tetrahydroxybenzophenon | 131-55-5 |

Schließlich sind auch mikronisierte Pigmente wie Titandioxid und Zinkoxid zu nennen.

Zum Schutz menschlicher Haare vor UV-Strahlen können die erfindugsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Für diesen Zweck besonders bevorzugte Verbindungen der Formel I sind solche, bei denen die Substituenten R³ und/oder R⁴ kationische Reste darstellen wie z.B. Verbindung 2 in Tabelle 3.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in wäßrigen oder alkoholisch-wäßrigen Systemen löslich und lassen sich somit leicht in die wäßrige Phase von kosmetischen Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

Die UV-Filterwirkung der erfindungsgemäßen Verbindungen der Formel I kann auch zur Stabilisierung von Wirk- und Hilfsstoffen in kosmetischen und pharmazeutischen Formulierungen ausgenutzt werden.

Gegenstand der Erfindung sind auch die Verbindungen der Formel I zur Verwendung als Medikament sowie pharmazeutische Mittel zur vorbeugenden Behandlung von Entzündungen und Allergien der Haut sowie zur Verhütung bestimmter Hautkrebsarten, welche eine wirksame Menge mindestens einer Verbindung der Formel I als Wirkstoff enthalten.

Das erfindungsgemäße pharmazeutische Mittel kann oral oder topisch verabreicht werden. Für die orale Verabreichung liegt das pharmazeutische Mittel in Form von u.a. Pastillen, Gelatinekapseln, Dragees, als Sirup, Lösung, Emulsion oder Suspension vor. Die topische Anwendung der pharmazeutischen Mittel erfolgt beispielsweise als Salbe, Creme, Gel, Spray, Lösung oder Lotion.

### Beispiele:

### I. Herstellung

### Beispiel 1:

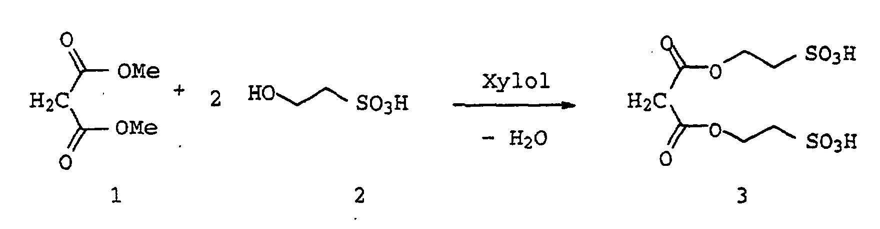

0,1 mol Malonsäuredimethylester (1) wurden mit 0,2 mol 2-Hydroxyethylsulfonsäure (2) in Xylol erhitzt. Das Reaktionswasser wurde laufend azeotrop entfernt. Nachdem das gesamte Reaktionswasser entfernt wurde, wurde die organische Phase mit 2 n NaOH gewaschen und das Wertprodukt (3) aus der wäßrigen Phase durch Ansäuern mit H₂SO₄ auf pH = 2 auskristallisiert. Ausbeute: 75 % d. Th.

### Beispiel 2:

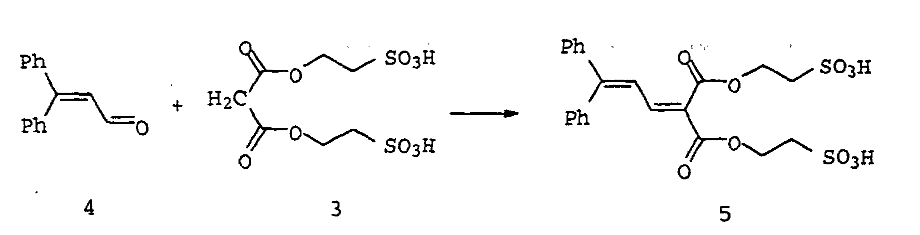

0,1 mol β-Phenylzimtaldehyd (4) wurden in 100 ml Xylol mit 0,11 mol der Verbindung 3 aus Beispiel 1 versetzt. Nach Zugabe von 0,01 mol Piperidin wurde das Reaktionsgemisch 4 h auf Rückfluß erhitzt. Nach Abkühlen wurde mit 2 n NaOH gewaschen, die wäßrige Phase nach Phasentrennung auf pH = 2 gestellt, wobei das Produkt (5) ausfiel. Ausbeute: 82 % d. Th. Reinheit: > 98% (HPLC). E¹₁ = 650, λₘₐₓ = 336 nm.

Die Herstellung der in Tabelle 3 aufgeführten Verbindungen erfolgte analog Beispiel 2.

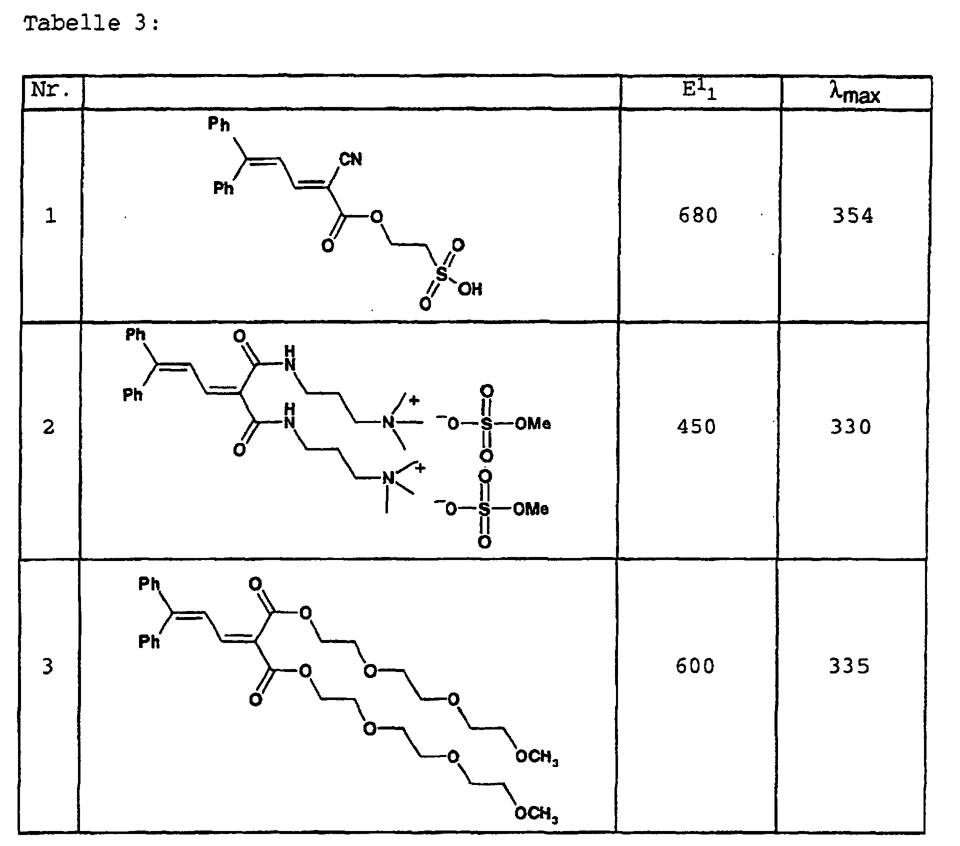

Analog Beispiel 2 oder wie im allgemeinen Teil beschrieben lassen sich die Verbindungen in den Tabellen 4 und 5 herstellen.

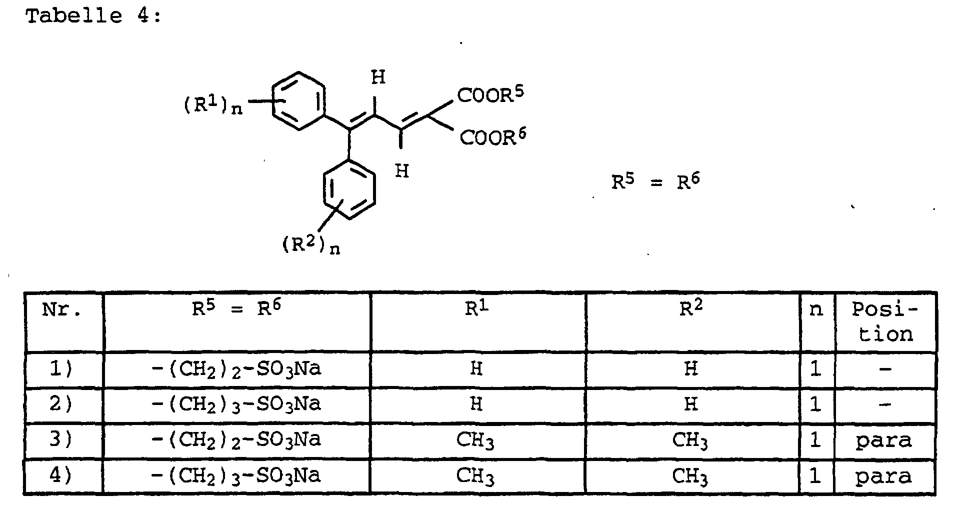

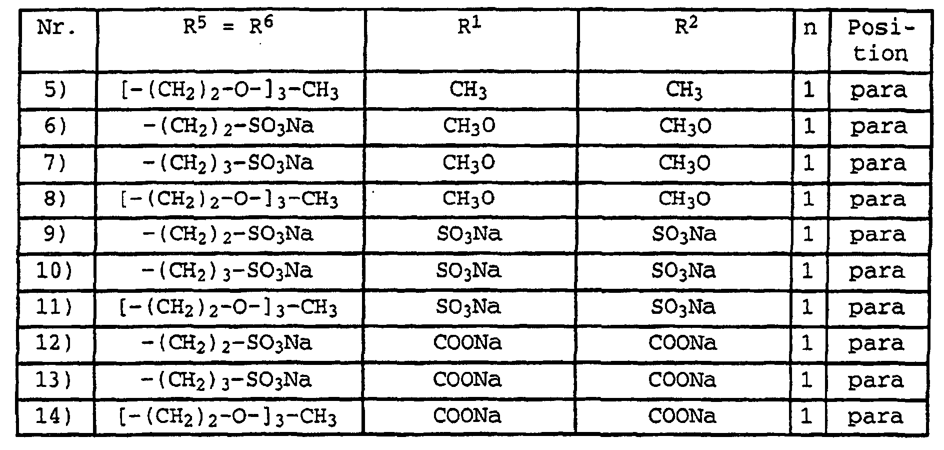

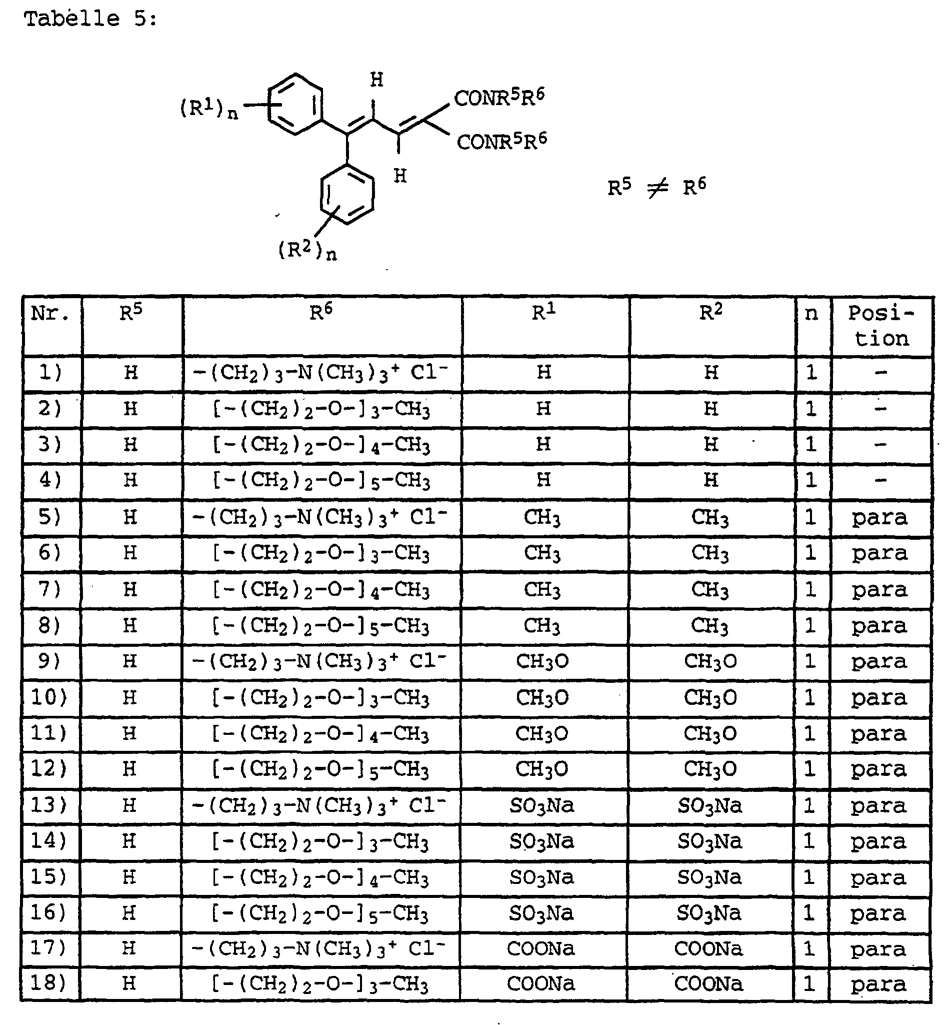

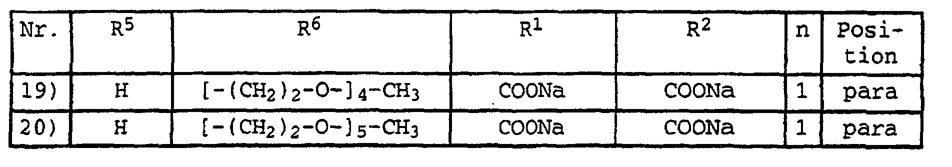

### Beispiel 4

### Standardisierte Methode zur Bestimmung der Photostabilität (Suntest)

Eine 5 Gew.-%ige alkoholisch-wäßrige Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampf zeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

Vergleichsversuche bez. Photostabilität:

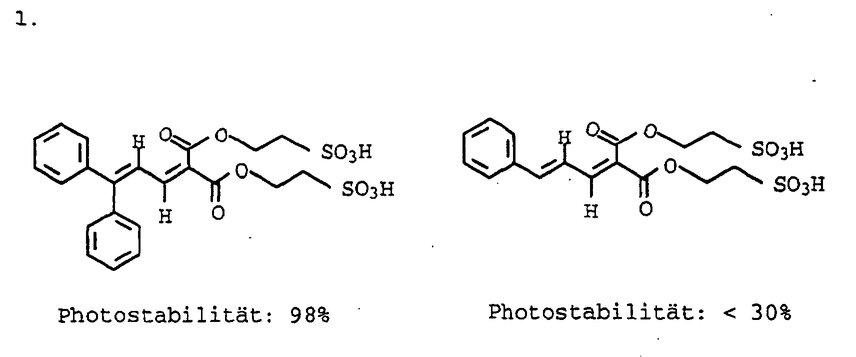

Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase, in der die erfindungsgemäßen 4,4-Diarylbutadiene der Formel I, gegebenenfalls in Form ihrer Salze, gelöst vorliegen unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 5

| Zusammensetzung für die Lippenpflege | |
|---|---|
| Massengehalt | |
| (Gew.-%) | |
| ad 100 | Eucerinum anhydricum |
| 10,00 | Glycerin |
| 10,00 | Titanium Dioxid |
| 5,00 | Verbindung aus Beispiel 2 |
| 8,00 | Octyl Methoxycinnamat |
| 5,00 | Zink Oxid |
| 4,00 | Castoröl |
| 4,00 | Pentaerythrithyl Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 6

| Zusammensetzung für die Lippenpflege | |
|---|---|
| Massengehalt | |
| (Gew.-%) | |
| ad 100 | Eucerinum anhydricum |
| 10,00 | Glycerin |
| 10,00 | Titanium Dioxid |
| 5,00 | Verbindung Nr. 1 der Tabelle 3 |
| 8,00 | Octyl Methoxycinnamat |
| 5, 00 | Zink Oxid |
| 4,00 | Castoröl |
| 4,00 | Pentaerythrithyl Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 7

| Zusammensetzung für Sunblocker mit Mikropigmenten | |
|---|---|
| Massengehalt | |
| (Gew.-%) | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung aus Beispiel 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 8

| Zusammensetzung für Sunblocker mit Mikropigmenten | |
|---|---|
| Massengehalt | |
| (Gew.-%) | |
| ad 100 | Wasser |
| 10,00 | Octyl Methoxycinnamat |
| 6,00 | PEG-7-Hydrogenated Castor Öl |
| 6,00 | Titanium Dioxid |
| 5,00 | Verbindung Nr. 1 der Tabelle 3 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 9

| Fettfreies Gel | |
|---|---|
| Massengehalt | |
| (Gew.-%) | |
| ad 100 | Wasser |
| 8,00 | Octyl Methoxycinnamat |
| 7,00 | Titanium Dioxid |
| 5,00 | Verbindung aus Beispiel 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinylurea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 10

| Fettfreies Gel | |
|---|---|
| Massengehalt | |
| (Gew.-%) | |
| ad 100 | Wasser |
| 8,00 | Octyl Methoxycinnamat |
| 7,00 | Titanium Dioxid |
| 5,00 | Verbindung Nr. 1 der Tabelle 3 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinylurea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 11

| Sonnencreme (LSF 20) | |
|---|---|
| Massengehalt | |
| (Gew.-%) | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung aus Beispiel 2 |
| 6,00 | Mineral Öl |
| 5,00 | Zink Oxid |
| 5,00 | Isopropyl Palmitat |
| 5,00 | Imidazolidinylurea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 12

| Sonnencreme (LSF 20) | |
|---|---|
| Massengehalt | |
| (Gew.-%) | |
| ad 100 | Wasser |
| 8,00 | Octyl Methoxycinnamat |
| 8,00 | Titanium Dioxid |
| 6,00 | PEG-7-Hydrogenated Castor Öl |
| 5,00 | Verbindung Nr. 1 der Tabelle 3 |
| 6,00 | Mineral Öl |
| 5,00 | Zink Oxid |
| 5,00 | Isopropyl Palmitat |
| 5,00 | Imidazolidinylurea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 13

| Sonnencreme wasserfest | |
|---|---|
| Massengehalt | |
| (Gew.-%) | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung aus Beispiel 2 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 14

| Sonnencreme wasserfest | |
|---|---|
| Massengehalt | |
| (Gew.-%) | |
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 1 der Tabelle 3 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 15

| Sonnenmilch (LSF 6) | |
|---|---|
| Massengehalt | |
| (Gew.-%) | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung aus Beispiel 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

### Beispiel 16

| Sonnenmilch (LSF 6) | |
|---|---|
| Massengehalt | |
| (Gew.-%) | |
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 1 der Tabelle 3 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von 4,4-Diarylbutadienen der Formel I, 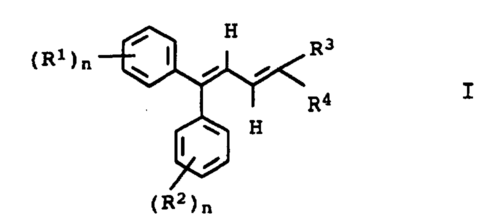 in der das Diensystem in der Z,Z; Z,E; E,Z oder E,E Konfiguration oder einer Mischung davon vorliegt und in der die Variablen unabhängig voneinander folgende Bedeutung haben:
R¹ und R² Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Heteroaryl, Aryl, das gegebenenfalls mit einem oder mehreren Resten wie Halogen, Cyano, Nitro, Amino, C₁-C₄-Alkylamino, C₁-C₄-Dialkylamino, Hydroxy, C₁-C₄-Alkyl, C₁-C₄-Alkoxy
oder anderen Resten substituiert sein kann,
wasserlöslich machende Substituenten, ausgewählt aus der Gruppe bestehend aus Carboxylat-, Sulfonat- oder Ammoniumresten;
R³ Wasserstoff, COOR⁵, COR⁵, CONR⁵R⁶, CN;
R⁴ COOR⁶, COR⁶, CONR⁵R⁶;
R⁵ Wasserstoff, [X]ₒ-R⁷, C₁-C₆-Alkylen-SO₃Y, C₁-C₆-Alkylen-PO₃Y, C₁-C₆-Alkylen-N(R⁸)₃⁺ A-;
R⁶ [X]ₒ-R⁷, C₁-C₆-Alkylen-SO₃Y, C₁-C₆-Alkylen-PO₃Y, C₁-C₆-Alkylen-N(R⁸)₃⁺ A-;
X -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
A Cl, Br, J, SO₄R⁹;
Y Wasserstoff, Na⁺, K⁺, Mg²⁺, Ca²⁺, Li⁺, Al³⁺, N(R⁸)₄⁺;
Z O, NH;
R⁷ und R⁸ Wasserstoff, C₁-C₆-Alkyl, C₂-C₆-Alkenyl, C₁-C₆-Acyl;
R⁹ Wasserstoff, C₁-C₆-Alkyl, C₂-C₆-Alkenyl;
n 1 bis 3;
o 1 bis 150
als wasserlösliche photostabile UV-Filter in kosmetischen und pharmazeutischen Zubereitungen zum Schutz der menschlichen Haut oder menschlicher Haare gegen Sonnenstrahlen, allein oder zusammen mit an sich für kosmetische und pharmazeutische Zubereitungen bekannten, im UV-Bereich absorbierenden Verbindungen.

2. Verwendung von Verbindungen der Formel I gemäß Anspruch 1 als wasserlösliche photostabile UV-A-Filter.

3. Verwendung von Verbindungen der Formel I gemäß den Ansprüchen 1 und 2 als wasserlösliche UV-Stabilisator in kosmetischen und pharmazeutischen Formulierungen.

4. Lichtschutzmittel enthaltende kosmetische und pharmazeutische Zubereitungen zum Schutz der menschlichen Epidermis oder menschlicher Haare gegen UV-Licht im Bereich von 280 bis 400 nm, **dadurch gekennzeichnet, daß** sie in einem kosmetisch und pharmazeutisch geeigneten Träger, allein oder zusammen mit an sich für kosmetische und pharmazeutische Zubereitungen bekannten im UV-Bereich absorbierenden Verbindungen, als photostabile UV-Filter wirksame Mengen von Verbindungen der Formel I 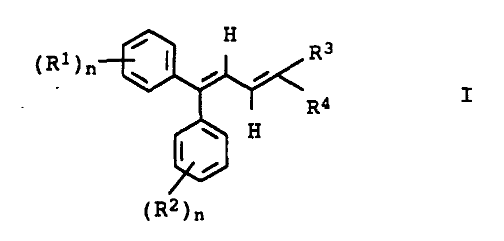 enthalten, in der die Variablen die Bedeutung gemäß Anspruch 1 haben.

5. 4,4-Diarylbutadiene der Formel I, 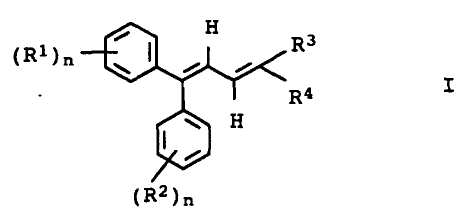 in der das Diensystem in der Z,Z; Z,E; E,Z oder E,E Konfiguration oder einer Mischung davon vorliegt und in der die Variablen unabhängig voneinander folgende Bedeutung haben:
R¹ und R² Wasserstoff, C₁-C₂₀-Alkyl, C₂-C₁₀-Alkenyl, C₃-C₁₀-Cycloalkyl, C₃-C₁₀-Cycloalkenyl, C₁-C₁₂-Alkoxy, C₁-C₂₀-Alkoxycarbonyl, C₁-C₁₂-Alkylamino, C₁-C₁₂-Dialkylamino, Heteroaryl, Aryl, das gegebenenfalls mit einem oder mehreren Resten wie Halogen, Cyano, Nitro, Amino, C₁-C₄-Alkylamino, C₁-C₄-Dialkylamino, Hydroxy, C₁-C₄-Alkyl, C₁-C₄-Alkoxy oder anderen Resten substituiert sein kann,
wasserlöslich machende Substituenten, ausgewählt aus der Gruppe bestehend aus Carboxylat-, Sulfonat- oder Ammoniumresten;
R³ Wasserstoff, COOR⁵, COR⁵, CONR⁵R⁶, CN;
R⁴ COOR⁶, COR⁶, CONR⁵R⁶;
R⁵ Wasserstoff, [X]ₒ-R⁷, C₁-C₆-Alkylen-SO₃Y, C₁-C₆-Alkylen-PO₃Y, C₁-C₆-Alkylen-N(R⁸)₃⁺ A-;
R⁶ [X]ₒ-R⁷, C₁-C₆-Alkylen-SO₃Y, C₁-C₆-Alkylen-PO₃Y, C₁-C₆-Alkylen-N(R⁸)₃⁺ A-;
X -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
A Cl, Br, J, SO₄R⁹;
Y Wasserstoff, Na⁺, K⁺, Mg²⁺, Ca²⁺, Li⁺, Al³⁺, N(R⁸)₄⁺;
Z O, NH;
R⁷ und R⁸ Wasserstoff, C₁-C₆-Alkyl, C₂-C₆-Alkenyl, C₁-C₆-Acyl;
R⁹ Wasserstoff, C₁-C₆-Alkyl, C₂-C₆-Alkenyl;
n 1 bis 3;
o 1 bis 150.

6. 4,4-Diarylbutadiene der Formel Ia nach Anspruch 5, 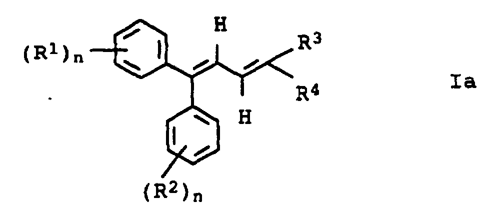 in der das Diensystem in der Z,Z; Z,E; E,Z oder E,E Konfiguration oder einer Mischung davon vorliegt und in der die Variablen unabhängig voneinander folgende Bedeutung haben:
R¹ und R² Wasserstoff, C₁-C₈-Alkyl, C₁-C₈-Alkoxy, wasserlöslich machende Substituenten, ausgewählt aus der Gruppe bestehend aus Carboxylat-, Sulfonat- oder Ammoniumresten;
R³ COOR⁵, CONR⁵R⁶, CN;
R⁴ COOR⁶, CONR⁵R⁶;
R⁵ Wasserstoff, [X]ₒ-R⁷, C₁-C₆-Alkylen-SO₃Y, C₁-C₆-Alkylen-N(R⁸)₃⁺ A-;
R⁶ [X]ₒ-R⁷, C₁-C₆-Alkylen-SO₃Y, C₁-C₆-Alkylen-N(R⁸)₃⁺ A-;
X -CH₂-CH₂-O-, -CH₂-CH₂-CH₂-O-, -CH(CH₃)-CH₂-O-;
A Cl, Br, J, SO₄R⁹;
Y Wasserstoff, Na⁺, K⁺, Mg²⁺, Ca²⁺, Li⁺, Al³⁺, N(R⁸)₄⁺;
R⁷ bis R⁹ Wasserstoff, C₁-C₃-Alkyl;
n 1 bis 3;
o 1 bis 50.

7. 4,4-Diarylbutadiene der Formel Ib nach Anspruch 5, 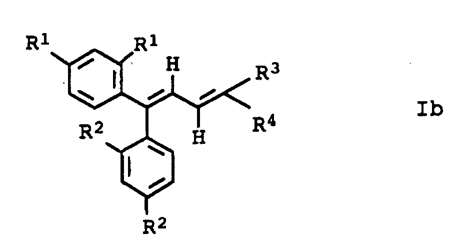 in der das Diensystem in der Z,Z; Z,E; E,Z oder E,E Konfiguration oder einer Mischung davon vorliegt und in der die Variablen unabhängig voneinander folgende Bedeutung haben:
R¹ und R² Wasserstoff, C₁-C₈-Alkyl, C₁-C₈-Alkoxy;
R³ COOR⁵, CONR⁵R⁶, CN;
R⁴ COOR⁶, CONR⁵R⁶;
R⁵ Wasserstoff, [X]ₒ-R⁷, C₁-C₆-Alkylen-SO₃Y, C₁-C₆-Alkylen-N(R⁸)₃⁺ A⁻;
R⁶ [X]ₒ-R⁷, C₁-C₆-Alkylen-SO₃Y, C₁-C₆-Alkylen-N(R⁸)₃⁺ A⁻;
X -CH₂-CH₂-O-, -CH₂-CH₂-CH₂-O-, -CH(CH₃)-CH₂-O-;
A Cl, Br, J, SO₄R⁹;
Y Wasserstoff, Na⁺, K⁺, Mg²⁺, Ca²⁺, Li⁺, Al³⁺, N(R⁸)₄⁺;
R⁷ bis R⁹ Wasserstoff, C₁-C₃-Alkyl;
o 1 bis 50.

8. 4,4-Diarylbutadiene der Formel Ic nach Anspruch 5, 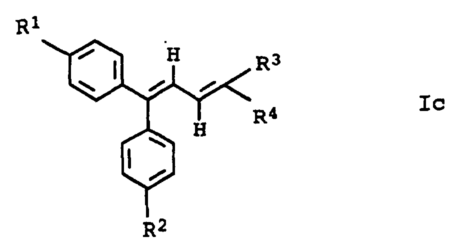 in der das Diensystem in der Z,Z; Z,E; E,Z oder E,E Konfiguration oder einer Mischung davon vorliegt und in der die Variablen unabhängig voneinander folgende Bedeutung haben:
R¹ und R² Wasserstoff, C₁-C₈-Alkyl, C₁-C₈-Alkoxy;
R³ COOR⁵, CONR⁵R⁶, CN;
R⁴ COOR⁶, CONR⁵R⁶;
R⁵ Wasserstoff, [X]ₒ-R⁷, C₁-C₆-Alkylen-SO₃Y, C₁-C₆-Alkylen-N(R⁸)₃⁺ A-;
R⁶ [X]ₒ-R⁷, C₁-C₆-Alkylen-SO₃Y, C₁-C₆-Alkylen-N(R⁸)₃⁺ A-;
X -CH₂-CH₂-O-, -CH₂-CH₂-CH₂-O-, -CH(CH₃)-CH₂-O-;
A Cl, Br, J, SO₄R⁹ ;
Y Wasserstoff, Na⁺, K⁺, Mg²⁺, Ca²⁺, Li⁺, Al³⁺, N(R⁸)₄⁺;
R⁷ bis R⁹ Wasserstoff, C₁-C₃-Alkyl;
o 1 bis 50.

## Claims

1. The use of 4,4-diarylbutadienes of the formula I, 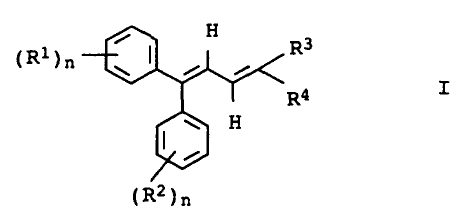 where the diene system has the Z,Z; Z,E; E,Z or E,E configuration or a mixture thereof, and where the variables independently of one another have the following meanings:
R¹ and R² hydrogen, C₁-C₂₀-alkyl, C₂-C₁₀-alkenyl, C₃-C₁₀-cycloalkyl, C₃-C₁₀-cycloalkenyl, C₁-C₁₂-alkoxy, C₁-C₂₀-alkoxycarbonyl, C₁-C₁₂-alkylamino, C₁-C₁₂-dialkylamino, heteroaryl, aryl, which may be unsubstituted by one or more radicals such as halogen, cycino, nitro, amino, C₁-C₄-alkylamino, C₁-C₄-dialkylamino, hydroxyl, C₁-C₄-alkyl, C₁-C₄-alkoxy or other radicals,
substituents which confer solubility in water and are selected from the group consisting of carboxylate, sulfonate or ammonium radicals;
R³ hydrogen, COOR⁵, COR⁵, CONR⁵R⁶, CN;
R⁴ COOR⁶, COR⁶, CONR⁵R⁶;
R⁵ hydrogen, [X]ₒ-R⁷, C₁-C₆-alkylene-SO₃Y, C₁-C₆-alkylene-PO₃Y, C₁-C₆-alkylene-N(R⁸)₃⁺ A⁻;
R⁶ [X]ₒ-R⁷, C₁-C₆-alkylene-SO₃Y, C₁-C₆-alkylene-PO₃Y, C₁-C₆-alkylene-N(R⁸)₃⁺ A-;
X -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
A Cl, Br, I, SO₄R⁹;
Y hydrogen, Na⁺, K⁺, Mg²⁺, Ca²⁺, Li⁺, Al³⁺, N(R⁸)₄⁺;
Z O, NH;
R⁷ and R⁸ hydrogen, C₁-C₆-alkyl, C₂-C₆-alkenyl, C₁-C₆-acyl;
R⁹ hydrogen, C₁-C₆-alkyl, C₂-C₆-alkenyl;
n 1 to 3;
o 1 to 150
as water-soluble photostable UV filters in cosmetic and pharmaceutical preparations for protecting the human skin or human hair from the sun's rays, alone or together with compounds which absorb in the UV region and are known per se for cosmetic and pharmaceutical preparations.

2. The use of compounds of the formula I as claimed in claim 1 as water-soluble photostable UV-A filters.

3. The use of compounds of the formula I as claimed in claims 1 and 2 as water-soluble UV stabilizers in cosmetic and pharmaceutical formulations.

4. A sunscreen-containing cosmetic or pharmaceutical preparation for protecting the human epidermis or human hair from UV light in the range from 280 to 400 nm, which comprises, in a cosmetically or pharmaceutically suitable carrier, alone or together with compounds which absorb in the UV region and are known per se for cosmetic and pharmaceutical preparations, amounts, which are effective as photostable UV filters, of compounds of the formula I 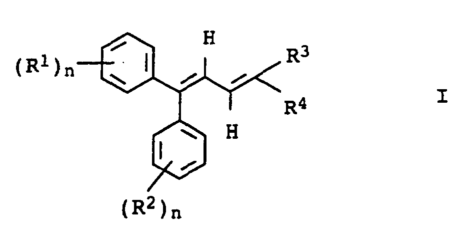 where the variables have the meanings stated in claim 1.

5. A 4,4-diarylbutadiene of the formula I, 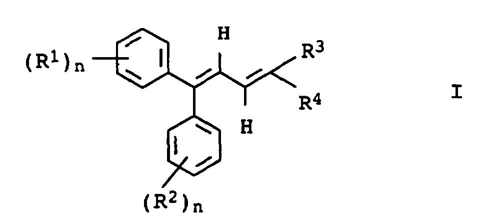 where the diene system has the Z,Z; Z,E; E,Z or E,E configuration or a mixture thereof, and where the variables independently of one another have the following meanings:
R¹ and R² hydrogen, C₁-C₂₀-alkyl, C₂-C₁₀-alkenyl, C₃-C₁₀-cycloalkyl, C₃-C₁₀-cycloalkenyl, C₁-C₁₂-alkoxy, C₁-C₂₀-alkoxycarbonyl, C₁-C₁₂-alkylamino, C₁-C₁₂-dialkylamino, heteroaryl, aryl, which may be unsubstituted by one or more radicals such as halogen, cycino, nitro, amino, C₁-C₄-alkylamino, C₁-C₄-dialkylamino, hydroxyl, C₁-C₄-alkyl, C₁-C₄-alkoxy or other radicals,
substituents which confer solubility in water and are selected from the group consisting of carboxylate, sulfonate or ammonium radicals;
R³ hydrogen, COOR⁵, COR⁵, CONR⁵R⁶, CN;
R⁴ COOR⁶, COR⁶, CONR⁵R⁶;
R⁵ hydrogen, [X]ₒ-R⁷, C₁-C₆-alkylene-SO₃Y, C₁-C₆-alkylene-PO₃Y, C₁-C₆-alkylene-N(R⁸)₃⁺ A-;
R⁶ [X]ₒ-R⁷, C₁-C₆-alkylene-SO₃Y, C₁-C₆-alkylene-PO₃Y, C₁-C₆-alkylene-N(R⁸)₃⁺ A-;
X -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z-;
A Cl, Br, I, SO₄R⁹;
Y hydrogen, Na⁺, K⁺, Mg²⁺, Ca²⁺, Li⁺, Al³⁺, N(R⁸)₄⁺;
Z O, NH;
R⁷ and R⁸ hydrogen, C₁-C₆-alkyl, C₂-C₆-alkenyl, C₁-C₆-acyl;
R⁹ hydrogen, C₁-C₆-alkyl, C₂-C₆-alkenyl;
n 1 to 3;
o 1 to 150.

6. A 4,4-diarylbutadiene of the formula Ia as claimed in claim 5. 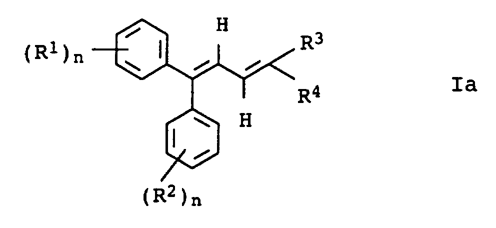 where the diene system has the Z,Z; Z,E; E,Z or E,E configuration or a mixture thereof, and where the variables independently of one another have the following meanings:
R¹ and R² hydrogen, C₁-C₈-alkyl, C₁-C₈-alkoxy, substituents which confer solubility in water and are selected from the group consisting of carboxylate, sulfonate or ammonium radicals;
R³ COOR⁵, CONR⁵R⁶, CN;
R⁴ COOR⁶, CONR⁵R⁶;
R⁵ hydrogen, [X]ₒ-R⁷, C₁-C₆-alkylene-SO₃Y, C₁-C₆-alkylene-N(R⁸)₃⁺ A-;
R⁶ [X]ₒ-R⁷, C₁-C₆-alkylene-SO₃Y, C₁-C₆-alkylene-N(R⁸)₃⁺ A⁻;
X -CH₂-CH₂-O-, -CH₂-CH₂-CH₂-O-, -CH(CH₃)-CH₂-O-;
A Cl, Br, I, SO₄R⁹;
Y hydrogen, Na⁺, R⁺, Mg²⁺, Ca²⁺, Li⁺, Al³⁺, N(R⁸)₄⁺;
R⁷ to R⁹ hydrogen, C₁-C₃-alkyl;
n 1 to 3;
o 1 to 50.

7. A 4,4-diarylbutadiene of the formula Ib as claimed in claim 5, 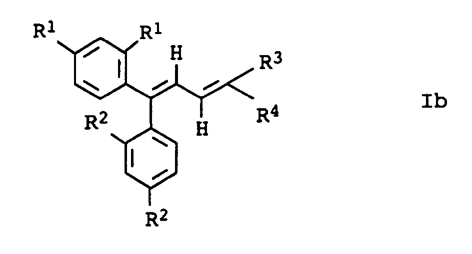 where the diene system has the Z,Z; Z,E; E,Z or E,E configuration or a mixture thereof, and where the variables independently of one another have the following meanings:
R¹ and R² hydrogen, C₁-C₈-alkyl, C₁-C₈-alkoxy;
R³ COOR⁵, CONR⁵R⁶, CN;
R⁴ COOR⁶, CONR⁵R⁶;
R⁵ hydrogen, [X]ₒ-R⁷, C₁-C₆-alkylene-SO₃Y, C₁-C₆-alkylene-N(R⁸)₃⁺ A-;
R⁶ [X]ₒ-R⁷, C₁-C₆-alkylene-SO₃Y, C₁-C₆-alkylene-N(R⁸)₃⁺ A-;
X -CH₂-CH₂-O-, -CH₂-CH₂-CH₂-O-, -CH(CH₃)-CH₂-O-;
A Cl, Br, I, SO₄R⁹;
Y hydrogen, Na⁺, K⁺, Mg²⁺, Ca²⁺, Li⁺, Al³⁺, N(R⁸)₄⁺;
R⁷ to R⁹ hydrogen, C₁-C₃-alkyl;
o 1 to 50.

8. A 4,4-diarylbutadiene of the formula Ic as claimed in claim 5, 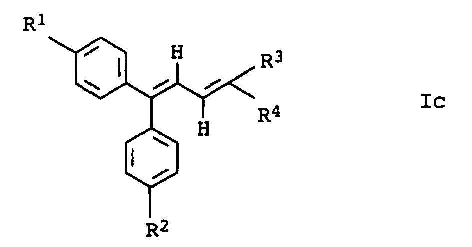 where the diene system has the Z,Z; Z,E; E,Z or E,E configuration or a mixture thereof, and where the variables independently of one another have the following meanings:
R¹ and R² hydrogen, C₁-C₈-alkyl, C₁-C₈-alkoxy;
R³ COOR⁵, CONR⁵R⁶, CN;
R⁴ COOR⁶, CONR⁵R⁶ ;
R⁵ hydrogen, [X]ₒ-R⁷, C₁-C₆-alkylene-SO₃Y, C₁-C₆-alkylene-N(R⁸)₃⁺ A-;
R⁶ [X]ₒ-R⁷, C₁-C₆-alkylene-SO₃Y, C₁-C₆-alkylene-N(R⁸)₃⁺ A-;
X -CH₂-CH₂-O-, -CH₂-CH₂-CH₂-O-, -CH(CH₃)-CH₂-O-;
A Cl, Br, I, SO₄R⁹;
Y hydrogen, Na⁺, K⁺, Mg²⁺, Ca²⁺, Li⁺, Al³⁺, N(R⁸)₄⁺;
R⁷ to R⁹ hydrogen, C₁-C₃-alkyl;
o 1 to 50.

## Revendications

1. Utilisation de 4,4-diarylbutadiènes de formule I 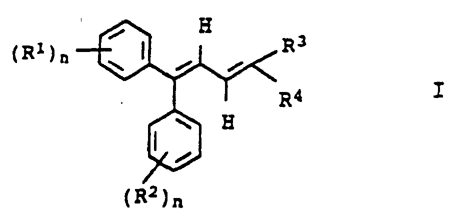 dans laquelle le système diénique est en configuration Z,Z ; Z,E ; E,Z ou E,E ou représente un mélange de ces diverses formes et les symboles de la formule ont, indépendamment les uns des autres, les significations suivantes :
R¹ et R²
l'hydrogène, des groupes alkyle en C1-C20, alcényle en C2-C10, cycloalkyle en C3-C10, cycloalcényle en C3-C10, alcoxy en C1-C12, (alcoxy en C1-C20)carbonyle, alkylamino en C1-C12, di-(alkyle en C1-C12)amino, hétéroaryle, aryle, lequel peut le cas échéant porter un ou plusieurs substituants tels que des halogènes, des groupes cyano, nitro, amino, alkylamino en C1-C4, di-(alkyle en C1-C4)amino, hydroxy, alkyle en C1-C4, alcoxy en C1-C4 ou d'autres encore,
des substituants hydrosolubilisants choisis parmi les groupes carboxylate, sulfonate et ammonium ;
R³ : l'hydrogène, un groupe COOR⁵, COR⁵, CONR⁵R⁶, CN ;
R⁴ : un groupe COOR⁶, COR⁶, CONR⁵R⁶ ;
R⁵ : l'hydrogène, un groupe [X]ₒR⁷ (alkylène en C1-C6)-SO₃Y, (alkylène en C1-C6)-PO₃Y, (alkylène en C1-C6)-N (R⁸)₃⁺A⁻;
R⁶ : un groupe [X]ₒ-R⁷ (alkylène en C1-C6)-SO₃Y, (alkylène en C1-C6)-PO₃Y, (alkylène en C1-C6)-N(R⁸)₃⁺ A⁻;
X : -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z- ;
A : Cl, Br, I, SO₄R⁹ ;
Y : l'hydrogène, Na⁺, K⁺, M_{g}⁺², Ca⁺², Li⁺, Al⁺³, N(R⁸)₄⁺ ;
Z : 0, NH ;
R⁷ et R⁸ ;
l'hydrogène, des groupes alkyle en C1-C6, alcényle en C2-C6, acyle en C1-C6;
R⁹ : l'hydrogène, un groupe alkyle en C1-C6, alcényle en C2-C6 ;
n: 1 à 3 ;
o : 1 à 150
en tant que filtres pour UV solubles dans l'eau et stables à la lumière dans des compositions cosmétiques et pharmaceutiques pour la protection de la peau humaine ou des cheveux humains contre les rayons solaires, seuls ou avec des composés absorbant dans le domaine UV connus en soi pour les compositions cosmétiques et pharmaceutiques.

2. Utilisation de composés de formule I de la revendication 1, en tant que filtres pour UV-A solubles dans l'eau et stables à la lumière.

3. Utilisation des composés de formule I selon les revendications 1 et 2 en tant que stabilisants contre les UV, solubles dans l'eau, dans des compositions cosmétiques et pharmaceutiques.

4. Compositions cosmétiques et pharmaceutiques contenant des agents de protection contre la lumière et prévus pour la protection de l'épiderme humain ou des cheveux humains contre la lumière UV dans l'intervalle de 280 à 400 nm, **caractérisées en ce qu'**elles contiennent, dans un véhicule approprié aux usages cosmétiques et pharmaceutiques, seuls ou avec des composés absorbant dans le domaine UV connus en soi pour des compositions cosmétiques et pharmaceutiques, des quantités efficaces en tant que filtres pour UV, stables à la lumière, de composés de formule I 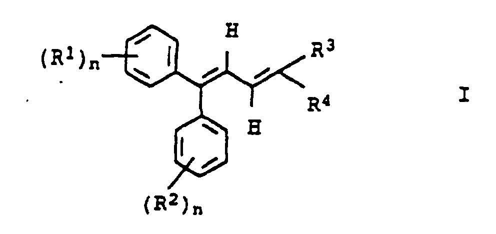 dans laquelle les symboles ont les significations indiquées dans la revendication 1.

5. 4,4-diarylbutadiènes de formule I 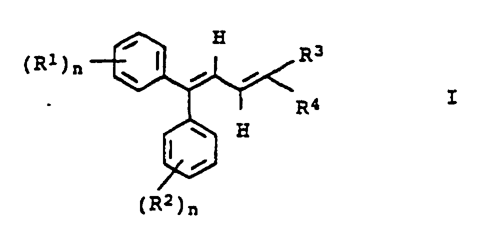 dans laquelle le système diénique est en configuration Z,Z ; Z,E ; E,Z ou E,E ou représente un mélange de ces configurations et les symboles ont, indépendamment les uns des autres, les significations suivantes
R¹ et R²;
l'hydrogène, des groupes alkyle en C1-C20, alcényle en C2-C10, cycloalkyle en C3-C10, cycloalcényle en C3-C10, alcoxy en C1-C12, (alcoxy en C1-C20)carbonyle, alkylamino en C1-C12, di-(alkyle en C1-C12)amino, hétéroaryle, aryle, lequel peut le cas échéant porter un ou plusieurs substituants tels que des halogènes, des groupes cyano, nitro, amino, alkylamino en C1-C4, di-(alkyle en C1-C4)amino, hydroxy, alkyle en C1-C4, alcoxy en C1-C4 ou d'autres encore,
des substituants hydrosolubilisants choisis parmi les groupes carboxylate, sulfonate, et ammonium ;
R³ : l'hydrogène, un groupe COOR⁵, COR⁵, CONR⁵R⁶, CN ;
R⁴ : un groupe COOR⁶, COR⁶, CONR⁵R⁶ ;
R⁵ : l'hydrogène, un groupe [X]ₒ-R⁷, (alkylène en C1-C6)-SO₃Y, (alkylène en C1-C6)-PO₃Y, (alkylène en C1-C6)-N(R⁸)₃ A⁻ ;
R⁶ : un groupe [X]ₒ-R⁷, (alkylène en C1-C6)-SO₃Y, (alkylène en C1-C6) PO₃Y, (alkylène en C1-C6)-N(R⁸)₃⁺ A⁻ ;
X : un groupe -CH₂-CH₂-Z-, -CH₂-CH₂-CH₂-Z-, -CH(CH₃)-CH₂-Z-, -CH₂-CH₂-CH₂-CH₂-Z-, -CH₂-CH(CH₂-CH₃)-Z- ;
A : Cl, Br, I, SO₄R⁹ ;
Y : l'hydrogène, Na⁺, K⁺, Mg⁺², Ca⁺², Li⁺, Al⁺³, N(R⁸)₄⁺ ;
Z : 0, NH ;
R⁷ et R⁸ :
l'hydrogène, des groupes alkyle en C1-C6, alcényle en C2-C6, acyle en C1-C6 ; R⁹ : l'hydrogène, un groupe alkyle en C1-C6, alcényle en C2-C6 ;
n : 1 à 3 ;
o : 1 à 150.

6. 4,4-diarylbutadiènes de formule la selon revendication 5; 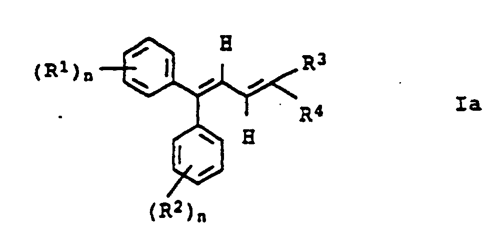 dans laquelle le système diénique est en configuration Z,Z ; Z,E ; E,Z ou E,E ou représentent un mélange de telles configurations et les symboles ont indépendamment les uns des autres, les significations suivantes
R¹ et R²,
l'hydrogène, des groupes alkyle en C1-C8, alcoxy en C1-C8,
des substituants hydrosolubilisants choisis parmi les groupes carboxylate, sulfonate et ammonium ;
R³ : un groupe COOR⁵, CONR⁵R⁶, CN ;
R⁴ : un groupe COOR⁶, CONR⁵R⁶ ;
R⁵ : l'hydrogène, un groupe [X]ₒ-R⁷ (alkylène en C1-C6)-SO₃Y, (alkylène en C1-C6)-N(R⁸)₃⁺ A⁻;
R⁶ : un groupe [X]ₒ-R⁷, (alkylène en C1-C6)-SO₃Y, (alkylène en C1-C6)-N(R⁸)₃⁺A⁻;
X : un groupe -CH₂-CH₂-O-, -CH ₂-CH₂-CH₂-O-, -CH(CH₃)-CH₂-O-;
A : Cl, Br, I, SO₄R⁹;
Y : l'hydrogène, Na⁺, K⁺, Mg⁺², Ca⁺², Li⁺, Al⁺³, N(R⁸)₄⁺;
R⁷ à R⁹
l'hydrogène, des groupes alkyle en C1-C3 ;
n: 1 à 3 ;
o : 1 à 50.

7. 4,4-diarylbutadiènes de formule Ib selon la revendication 5, 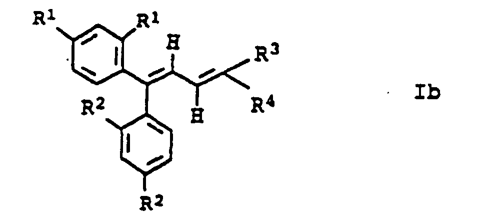 dans laquelle le système diénique est en configuration Z,Z ; Z,E ; E,Z ou E,E ou représente un mélange de telles configurations et les symboles ont, indépendamment les uns des autres, les significations suivantes
R¹ et R^{2,}
l'hydrogène, des groupes alkyle en C1-C8, alcoxy en C1-C8 ;
R³ : un groupe COOR⁵, CONR⁵R⁶, CN ;
R⁴ : un groupe COOR⁶, CONR⁵R⁶ ;
R⁵: l'hydrogène, un groupe [X]ₒ-R⁷, (alkylène en C1-C6)-SO₃Y, (alkylène en C1-C6)-N(R⁸)₃⁺ A⁻;
R⁶ : un groupe [X]ₒ-R⁷, (alkylène en C₁-C₆)-SO₃Y, (alkylène en C1-C6)-N(R⁸)₃⁺ A⁻ ;
X : un groupe -CH ₂-CH₂-O-, -CH₂-CH₂-CH₂-O-, -CH(CH₃)-CH₂-O- ;
A : Cl, Br, I, SO₄R⁹ ;
Y. l'hydrogène, Na⁺, K⁺, Mg⁺², Ca⁺², Li⁺, Al⁺³, N(R⁸)₄⁺ ;
R⁷ à R⁹,
l'hydrogène, des groupes alkyle en C1-C3 ;
o : 1 à 50.

8. 4,4-diarylbutadiènes de formule le selon revendication 5, 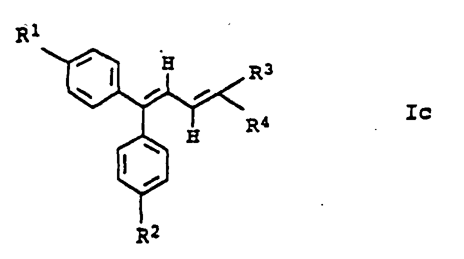 dans laquelle le système diénique est en configuration Z,Z ; Z,E ; E,Z ou E,E ou représente un mélange de telles configurations, et les symboles ont, indépendamment, les uns des autres, les significations suivantes :
R¹ et R²
l'hydrogène, des groupes alkyle en C1-C8, alcoxy en C1-C8 ;
R³ : un groupe COOR⁵, CONR⁵R⁶, CN ;
R⁴ : un groupe COOR⁶, CONR⁵R⁶ ;
R⁵ : l'hydrogène, un groupe [X]ₒ-R⁷, (alkylène en C1-C6)-SO₃Y, (alkylène en C1-C6)-N(R⁸)₃⁺ A⁻;
R⁶ : un groupe [X]ₒ-R⁷ ;(alkylène en C1-C6)-SO₃Y, (alkylène en C1-C6)-N(R⁸)₃⁺A⁻;
X : un groupe -CH ₂-CH₂-O-, -CH ₂-CH₂-CH₂-O-, -CH(CH₃)-CH₂-O- ;
A : Cl, Br, I, SO₄R⁹ ;
Y : l'hydrogène, Na⁺, K⁺, Mg⁺², Ca⁺², Li⁺, Al⁺³, N(R8)₄⁺ ;
R⁷ à R⁹
l'hydrogène, des groupes alkyle en C1-C4 ;
o : 1 à 50.
